# EUROPEAN PATENT SPECIFICATION

(11) **EP 2 911 703 B1**
(45) Date of publication and mention of the grant of the patent: **03.06.2020**
(21) Application number: 13783308.3
(22) Date of filing: 23.10.2013
(51) Int. Cl.: A61K 49/00, A23L 33/00, A23L 33/15, A23L 33/16, A23L 33/17

(54) **SYSTEM FOR USE IN DYSPHAGIA DIAGNOSIS**
SYSTEM ZUR VERWENDUNG IN DER DYSPHAGIEDIAGNOSE
SYSTÈME DESTINÉ À ÊTRE UTILISÉ DANS UN DIAGNOSTIC DE DYSPHAGIE

(30) Priority: 26.10.2012 EP 12190225
(43) Date of publication of application: 02.09.2015
(73) Proprietor: Fresenius Kabi Deutschland GmbH, 61352 Bad Homburg (DE)
(72) Inventor: MARTIN, Wendy, 60311 Frankfurt (DE); BRITO DE LA FUENTE, Edmundo, 61381 Friedrichsdorf (DE); GALLEGOS-MONTES, Crispulo, 61352 Bad Homburg (DE); ICKENSTEIN, Guntram, 01309 Dresden (DE)
(74) Representative: Fresenius Kabi Deutschland GmbH
(86) International application number: PCT/EP2013/072173
(87) International publication number: WO 2014/064154

(56) References cited:
- WO-A1-2011/152706
- WO-A1-2013/135737
- US-A- 5 976 084
- CASANOVAS A ET AL: "Cluster classification of dysphagia-oriented products considering flow, thixotropy and oscillatory testing", FOOD HYDROCOLLOIDS, ELSEVIER BV, NL, vol. 25, no. 5, 30 July 2010 (2010-07-30), pages 851-859, XP028163224, ISSN: 0268-005X, DOI: 10.1016/J.FOODHYD.2010.07.029 [retrieved on 2010-08-20]

## Description

### FIELD OF THE INVENTION

The present disclosure is concerned with a system for use in dysphagia diagnosis and nutrition.

### BACKGROUND OF THE INVENTION

Dysphagia is a common health problem describing a range of mechanical disorders that affect the safety, efficiency, or quality of eating and drinking. In dysphagia, every swallowing attempt bears the danger of choking and aspiration of liquids or food particles into the lung. Hence, dysphagia patients have an increased risk of developing aspiration pneumonia, which may even lead to death. Furthermore, dehydration, malnutrition, and often a reduced quality of life are direct consequences of dysphagia.

Management of dysphagia comprises the prescription of so called texture-controlled diets. Therefore, diagnosis procedures aim to define the degree / kind of swallowing difficulty and are used to find out which texture the patient can safely swallow. Thus, the textures a patient is able to swallow follow from an assessment of the swallowing problem.

Currently, diagnosis of dysphagia typically comprises having the patient swallow test samples. The actual diagnosis may then be done by simple clinical assessments, e.g. a health care professional (HCP) observes the swallowing ability of the patient e.g. the tone of his voice or the movements of the swallowing tract. There are many different protocols for a clinical assessment and thus aspiration can go undetected. On the other hand, diagnosis may be done by instrumental diagnosis, such as endoscopy (FEES) or videofluoroscopic swallowing study (VFSS, an x-ray procedure). As a result of any of the diagnostic methods, a "food texture" which has been swallowed safely and efficiently during diagnosis and later as nutrition to the patient is recommended

The diagnosis methods typically have in common that the patient has to swallow test samples, such as yoghurt. However, even without specific focus on dysphagia, it is generally known that, e.g. depending on the chosen brand, swallowing of yoghurt feels different for different yoghurts. They may feel "thicker", i.e. harder to swallow, or thinner, i.e. easier to swallow. Thus depending on the accidental choice of yoghurt brand, a patient may or may not be diagnosed with the ability to swallow yoghurt like food textures safely.

Moreover, depending on the HCPs personal protocol for preparing test samples and the nature of the samples used, textures - such as "honey like" - may be considered safe by one HCP at one specific facility, while another HCP at another facility may only allow "pudding like" for the same patient. It has even been suggested that HCPs are rarely able to reproduce the same texture twice (Glassburn, D. Dysphagia 13:218-222, 1998). It appears that currently, there is no clear guideline for HCP to achieve this.

US 5 976 084 A discloses a method and a kit for the evaluation of the severity of dysphagia. The kit contains samples of materials to be swallowed by the patients with standardized properties for various parameter. The specific severity of the dysphagia can be evaluated and identified for each specific patient based upon the level of each parameter. By charting of the responses to each and every one of the parameters, a more clinical valuation of the specific degree of dysphagia problem can be identified and used in a more precise evaluation of appropriate forms of foods for dietary nourishment.

In VFSS, the test samples have additionally to comprise contrast agents such as BaSO₄ for making the swallowing process visible under x-ray. Generally, the addition of contrast agents, such as BaSO₄, to test samples is believed to have a substantial influence on the rheological properties of such a sample. Moreover, such samples are for testing only and cannot be used in products for continuous patient nutrition because of posing a considerable health risk.

In particular with regard to dysphagia diagnosis endoscopy, wherein typically food test samples are prepared by an HCP, one drawback is that they are only vaguely defined. Thus, there is a need to improve comparability and/or reproducibility of swallowing assessment (Glassburn et al., Dysphagia 13:218-222, 1998).

Moreover, the swallowing mechanism exerts forces to swallowed test samples. However, typically test samples only defined by their "texture", i.e. may be Newtonian, shear thickening or shear thinning to a not known degree. Thus, a honey like consistency of test sample A may be safely swallowed, while a honey like viscosity of test sample B may cause choking. In such a case, the recommended texture may be severely influenced by or will depend on the selection of the test sample.

Also, after a safe texture has been determined by dysphagia diagnosis, it is typically aimed to reproduce this texture for the patient's daily food and drinks. This poses an additional risk for providing a patient with a product he is not able to swallow e.g. in cases wherein simply the definitions of textures are not comparable. It has also been reported that texture modified diets vary greatly from the test substances (Cichero, J., Dysphagia 15:188-200, 2000). In addition, the attempts to reach a certain texture often make the meal unpalatable or unappealing. This leaves the patient with food and drinks of unreliable texture and puts all goals of dysphagia therapy into question.

While for some dysphagia patients, or their personal care takers, it may be possible to learn to prepare foods that the patient can safely swallow; there is still a need to provide them with nourishment options for occasions where they or their care takers are hindered from preparing their food, when they are travelling, and the like.

Accordingly there is a general need for improved dysphagia diagnosis and dysphagia dietary management.

### SUMMARY OF THE INVENTION

In a first aspect the present invention relates to a system for use in diagnosis of dysphagia and nutrition of dysphagia patients according to the claims. For example, the diagnosis may be endoscopic diagnosis of dysphagia. The system comprises at least a first, a second and a third enteral nutrition product having each
a) a defined nutrient profile balanced for complete enteral nutrition or for supplemental enteral nutrition;
b) a shear rate dependent viscosity profile, wherein at each shear rate value between 1-100 s⁻¹ the following condition is fulfilled:
   viscosity product 1 > viscosity product 2 > viscosity product 3.

It is disclosed that at least one of such enteral nutrition products may be used for continuous nourishment of dysphagia patients which have been diagnosed by use of the system according to the first aspect of the invention.

The present disclosure relates also to a product line-up for use in diagnosis of dysphagia and simultaneous use in continuous nourishment of dysphagia patients comprising a first set of enteral nutrition products, a second set of enteral nutrition products and a third set of enteral nutrition products and optionally a fourth set of enteral nutrition products, wherein the products within a set are characterized by
a. a defined nutrient profile, wherein the nutrient profile is balanced for complete enteral nutrition or for supplemental enteral nutrition;
   and wherein
b. each product has a shear rate dependent viscosity profile, wherein at each shear rate value between 1-100 s⁻¹ the following condition is fulfilled:
viscosity of any product belonging to the first set of products > viscosity of any product belonging to the second set of products > viscosity of any product belonging to the third set of products and, if the product line-up comprises a fourth set of products > viscosity of any product belonging to the third set of products.

Further exemplary aspects and embodiments of the invention will become apparent to a person skilled in the art from the below. Preferred embodiments are listed in the dependent claims.

### DETAILED DESCRIPTION OF THE INVENTION

"Dysphagia" as used herein refers to abnormalities, such as difficulties, in the swallowing process.

"Texture" herein refers to the viscosity of a food product of an enteral nutrition product as defined by assessment of viscosity at a single shear rate (e.g. at 1, or 50 or 100 s⁻¹). Accordingly, from a texture the viscosity profile is not known. E.g. from the texture of a product, it is not possible to distinguish between Newtonian, shear thinning and shear thickening fluids.

"Viscosity profile" as used herein refers to the shear rate dependent viscosity of a product. A viscosity profile can be defined by determining viscosities at at least 2 different shear rates, preferably at least 3 different shear rates, more preferred at least 4 different shear rates, even more preferred, the profile is an almost continuous curve over the given shear rate range.

"Enteral nutrition product" as used herein refers to a ready to use synthetically produced food composition. Nutritional products as used herein typically are provided in fluid form, such as the drinks, crèmes or gels. Thus, nutritional products are artificial products obtained by mixing / dissolving ingredients whereby said ingredients are typically provided in solid form (e.g. powders) or liquid from. The term "nutrition product" excludes non-modified natural foods, such as for example meat, vegetables, fruit in their natural from and conventionally prepared (e.g. cooked) meals as well as conventional food, such as puréed food and yoghurt and the like. In contrast to conventional food products, enteral nutrition products have controlled constant composition with respect to their ingredients and nutrient profile, whereby actual control of a patient's diet is enabled. Typically, the enteral nutrition products herein are ready to use.

"Ready to use" as used herein refers to the final from of the enteral nutrition product as served to a patient or any other subject in need thereof.

"Enteral nutrition product balanced for complete enteral nutrition" refers to an enteral nutrition product having a nutrient profile which is balanced for complete enteral nutrition and suitable as sole source of nutrition. Such a nutrient profile comprises predefined amounts of energy (kilocalories) per unit mass or volume, macronutrients, such as protein, fat and carbohydrates as well as micronutrients, such as vitamins, minerals and trace elements which are essential for body functioning. An example of vitamins and minerals and recommended amounts for complete enteral nutrition can be found in EU directive 1999/21/EC Annex, Table 2. The term "enteral nutrition product balanced for complete enteral nutrition" as used herein generally corresponds to "nutritionally complete foods" as defined by EU directive 1999/21/EC Article 1, paragraph 3 (a) and (b).

"Enteral nutrition product balanced for supplemental enteral nutrition" refers to an enteral nutrition product having a nutrient profile which comprises predefined amounts of single or combined macro- and/or micronutrients, such as protein and/or fat and/or carbohydrates and/or vitamins and/or minerals and/or trace elements. The product composition is, however, not suitable as a sole source of nutrition. Thus, even when it is possible to consume a recommended daily amount of calories in from of such a product an under supply of one or more nutrients will occur. The term "enteral nutrition product balanced for supplemental enteral nutrition" as used herein generally corresponds to "nutritionally incomplete foods" as defined by EU directive 1999/21/EC Article 1, paragraph 3 (c).

### Swallowing mechanism (in humans)

Swallowing is a complex mechanism using both skeletal muscle (tongue) and smooth muscles of the pharynx and esophagus. In particular, swallowing comprises three phases
- an oral phase
- a pharyngeal phase and
- an esophageal phase.

Each phase is controlled by a different (neurological) mechanism. The autonomic nervous system coordinates this process in the pharyngeal and esophageal phases.

The **oral phase** is voluntary and mainly controlled by the medial temporal lobes and limbic system of the cerebral cortex with contributions from the motor cortex and other cortical areas. The oral phase comprises the formation of the bolus. Herein, bolus refers to food being ready for entering the pharyngeal phase. During bolus formation, food is contacted with saliva from the salivary glands and chewed (i.e. broken down by the teeth). This part of the swallowing process, in particular the bolus formation is significantly affected by material flow properties, i.e. viscosity. When ready for swallowing, the bolus will be moved towards the back of the tongue.

Once the bolus reaches the palatoglossal arch of the oropharynx, the pharyngeal phase, which is a reflex often also referred to as gag reflex begins. The reflex is initiated by touch receptors in the pharynx as a bolus of food is pushed to the back of the mouth by the tongue and subsequently is coordinated by the swallowing center in the medulla oblongata and pons. For the pharyngeal phase to work properly all other egress from the pharynx must be occluded - this includes the nasopharynx and the larynx. When the pharyngeal phase begins, other activities such as chewing, breathing, coughing and vomiting are concomitantly inhibited.

At the end of the **pharyngeal phase** the bolus moves down towards the esophagus by pharyngeal peristalsis which takes place by sequential contraction of the superior, middle and inferior pharyngeal constrictor muscles. The velocity through the pharynx depends on a number of factors such as viscosity and volume of the bolus.

Like the pharyngeal phase of swallowing, the **esophageal phase** of swallowing is under involuntary neuromuscular control. However, propagation of the food bolus is slower than in the pharynx. The bolus enters the esophagus and is propelled downwards first by striated muscle (recurrent laryngeal then by the smooth muscle. The upper esophageal sphincter relaxes to let food pass, after which various striated constrictor muscles of the pharynx as well as peristalsis and relaxation of the lower esophageal sphincter sequentially push the bolus of food through the esophagus into the stomach.

Each of the phases referred to above imparts certain mechanical forces to the food or nutritional product to be swallowed.

### Dysphagia

Abnormalities in swallowing in any of the above described phases will be referred to as dysphagia. Dysphagia is typically developed as a consequence of weakness or complete lack of coordination in the mouth and throat muscles. More specifically, abnormalities of the pharynx and/or oral cavity may lead to oropharyngeal dysphagia, whereas abnormalities of the esophagus may lead to esophageal dysphagia.

Typical causes of dysphagia are stroke, motor neuronal diseases, Parkinson's disease, cerebral palsy, head injuries, multiple sclerosis, surgery to the head and neck. For example, swallowing becomes a great concern for the elderly since strokes and Alzheimer's disease can interfere with the autonomic nervous system. However, dysphagia is a symptom of many diseases. It has therefore to be understood that abnormalities in swallowing are not necessarily limited to elderly patients.

### Diagnosis and management of dysphagia

Generally, patients suffering from dysphagia are at an increased risk of impaired nutritional status, aspiration pneumonia and dehydration. Dysphagia affects the safety and efficiency of food ingestion. In patients suffering from dysphagia, safety of swallowing is impaired: e.g. food going down the "wrong way" (aspiration), i.e. into the respiratory tract instead of the gastrointestinal tract, creates a substantial risk of aspiration pneumonia. Moreover, the efficiency of swallowing is impaired: when not enough food reaches the gastrointestinal tract for nutrition purposes which poses the risk of malnutrition. Thus, goals of dysphagia management typically are to avoid aspiration and malnutrition, while food intake, health and well-being shall be fostered.

This is often tried to achieve by modifying the texture of food, namely using pureed and/ or thickened foods. However, thickened and / or purred foods do not have a controlled viscosity profile, thus - even though decreased, there remains a risk of choking even though the prescribed texture is provided.

The extent of the swallowing problem as well as the "textures" of food a patient may be able to swallow can be diagnosed by endoscopic diagnosis.

Endoscopic diagnosis is will be carried out according to hospital protocols, including the addition of colorants as needed.

### System for diagnosis of dysphagia and exemplary advantages thereof

The system for use in diagnosis of dysphagia and nutrition of dysphagia patients according to the present disclosure comprises at least a first, a second and a third enteral nutrition product having each
a. a defined nutrient profile balanced for complete enteral nutrition and / or for supplemental enteral nutrition;
b. a shear rate dependent viscosity profile, wherein at each shear rate value between 1-100 s⁻¹ the following condition is fulfilled: viscosity product 1 > viscosity product 2 > viscosity product 3.

In a preferred embodiment, the system is used in endoscopic diagnosis, such as FEES. Typically, the enteral nutrition products of the system which has been swallowed safely will then also be used as patient nutrition. Alternatively, a product from the same set of products as defined below will be recommended.

The system may further comprising a fourth enteral nutrition product having a nutrient profile balanced for complete enteral nutrition and a shear rate dependent viscosity profile, wherein at each shear rate value between 1 and 100 s⁻¹ fulfils the following condition:
viscosity product 3 > viscosity product 4. This is believed to improve diagnosis and nutrition by expanding the rheological space covered by the system.

In some embodiments, each product comprised by the system has a shear thinning viscosity profile. Thereby, the comparability to many foods may be improved.

The viscosity of the at least three enteral nutrition products comprised by a system will typically be presented in a log-log scale. In some embodiments, the viscosity profiles of the at least three enteral nutrition products in a log-log scale are essentially parallel.

Typically, the viscosity of each of the products at 1 s⁻¹ lies between 10⁻²-10³ Pas.

The viscosity of the first product at 1 s⁻¹ may be lower than 10³ and higher than 10² Pas.

The viscosity of the second product at 1 s⁻¹ may be lower than 10² and higher than 10¹ Pas.

The viscosity of the third product at 1 s⁻¹ may be lower than 10¹ and higher than 10° Pas.

Preferably, there is a defined gap between the viscosities of the first, second, third and optional third product. For example, the viscosity of the first product at 1 s⁻¹ is at least 10 Pas higher than the viscosity of the second product.

On advantage of the system herein is that the same enteral nutrition products can be used for diagnosis and complete nutrition. Thus, draw backs resulting from differences between samples used in diagnosis and daily nutrition as well as from lack of standardization are improved simultaneously. There is no variability in the transfer from diagnosis to nutrition.

Also, the system simplifies procedures in medical care and / or hospitals, where diagnosis of dysphagia and preparation of nutrition for patients are typically conducted by different people and thus need to be communicated and coordinated in a reliable manner which may require following strict protocols for meal preparation. The latter, as well as the preparation of test samples by an HCP being in rather time consuming. Thus, the system described herein advantageously.

In view of offering several predefined viscosity profiles, the system herein covers a significant rheological space and, when used not only in diagnosis, but also as patient nutrition, prevents swallowing difficulties associated with insufficient knowledge and/or definition of a viscosity profile.

Without whishing to be bound by theory, it is believed that a viscosity profile which is defined in a sear rate range of 1- 100 s⁻¹ already offers an improved model for the forces exerted during the relevant stages of the swallowing process. Even more reliable results are expected for a viscosity profile which is defined in a sear rate range of 1- 1000 s⁻¹ (Brito et al., Applied Rheology 22, 53365, 2012).

The reproducibility and comparability of FEES exams is increased. Results can be compared to each other (e.g. to see the patient development or to compare different patients, or for a study) because the viscosity profiles within the system are defined and within a set of products the same.

Compared to test samples prepared from normal food, which may differ from preparation to preparation, by using the present system it can be assessed with increased accuracy whether the swallowing ability of a patient has changed compared to his last FEES exam.

The patient can feel reassured that they receive the correct texture, which spares them unpleasant and dangerous episodes of choking.

### Product line-up

The present disclosure also relates to a product line-up for use in diagnosis of dysphagia and simultaneous use in continuous nourishment of dysphagia patients comprising a first set of enteral nutrition products, a second set of enteral nutrition products and a third set of enteral nutrition products wherein the products within a set are characterized by
a. the same defined nutrient profile, wherein the nutrient profile is balanced for complete enteral nutrition or for supplemental enteral nutrition;
   and wherein
b. each product has a shear rate dependent viscosity profile, wherein at each shear rate value between 1-100 ; s⁻¹ the following condition is fulfilled:
   viscosity of any product belonging to the first set of products > viscosity of any product belonging to the second set of products > viscosity of any product belonging to the third set of products.

In one embodiment, each set of products comprises at least two products having a difference in overall composition.

In order to offer a variety for the patient, each set of products said difference in overall composition may for example be achieved by at least two products comprising different aroma. Exemplary suitable aromas are listed below.

In addition or alternatively, the included micronutrients may differ within a set of products in composition and / or their respective amounts.

### Enteral nutrition products

Exemplary enteral nutrition products balanced for complete enteral nutrition should comprise
a. predefined amounts of energy (kilocalories), typically per unit volume,
b. predefined amounts of macronutrients, such as protein, fat and carbohydrates and
c. predefined amounts of micronutrients, such as vitamins, minerals and trace elements which are essential for body functioning.

An example of vitamins and minerals and recommended amounts for complete enteral nutrition can be found in EU directive 1999/21/EC Annex, Table 2.

Enteral nutrition products balanced for supplemental enteral nutrition comprise predefined amounts of single or combined macro- and/or micronutrients, such as protein and/or fat and/or carbohydrates and/or vitamins and/or minerals and/or trace elements. The product composition is, however, not suitable as a sole source of nutrition. Thus, even when it is possible to consume a recommended daily amount of calories in from of such a product an under supply of one or more nutrients will occur.

For example, an enteral nutrition product balanced for supplemental enteral nutrition may comprise one or more, but not all of the vitamins and minerals in which can be found in EU directive 1999/21/EC Annex, Table 2. In another example an enteral nutrition product balanced for supplemental enteral nutrition may comprise one or more or all of the vitamins and minerals in which can be found in EU directive 1999/21/EC Annex, Table 2, however, but not all of them are provided in their recommended amounts for complete enteral nutrition.

Typically, proteins are included in the enteral products as amino acid source. The proteins included may be from different sources such as vegetable or fat sources. Suitable proteins can be selected from the list consisting of milk protein, such as casein, whey protein, soy protein, pea protein and hydrolisates thereof. Amino acids may as well be added in their chemical from or in the form of peptides.

Fat from different sources, such as animal and vegetable fat, may be included in the enteral nutrition product. The fat may include fatty acids, such as polyunsaturated fatty acids, monounsaturated fatty acids and polyunsaturated fatty acids. Suitable fatty acids may be selected form the group consisting of caproic acid (C6:0), caprylic acid (C8:0), capric acid (C10:0), lauric acid (C12:0), myristic acid (C14:0), palmitic acid (C16:0), palmitoleic acid (C16:1w7), stearic acid C18:0, oleic acid (C181:1w9), linoleic acid (C18:2w6), a-Linolenic acid (C18:3w3), eicosapentaenoic acid (C20:5w3), docosahexaenoic acid C22:6w3 and mixtures thereof. In addition to said fatty acids, C6-C12 medium chain triglycerides may be added.

Typical carbohydrate sources may be selected from the list consisting of maltodextirine, glucose syrup, sucrose, starch, isomaltolose, fructose and mixtures thereof.

In addition, the enteral nutrition product herein may comprise ingredients specifically as dietary fibres. Suitable dietary fibres may be selected from the group consisting of cocoa powder, inulin, wheat dextrine, cellulose, microcrystalline cellulose, soy polysaccharides, tapioca dextrine, xanthan, fructooligosaccharides, galactooligosaccharides, at least partially hydrolized guar gum, acacia gum, pectin, oat fibre, poly dextrose, resistant starch, hemicellulose and mixtures thereof.

An enteral nutrition product according to the present disclosure may for example comprise water, sucrose, milk protein, vegetable oils, emulsifier (such as soya lecithin), maltodextrin, inulin (for example obtained from chicory), vitamins, mineral and trace elements in predefined amounts. In addition thickeners and optional ingredients as specified below may be added.

The enteral nutrition products will typically be provided in an oil-in-water emulsion (O/W). Such an emulsion may be adapted to have an energy content of 0.5-5kcal/mL, preferably between 1 and 3kcal/mL.

Suitable thickeners to adapt the viscosity may be food thickeners which frequently are based on either polysaccharides (starches, vegetable gums, and pectin), or proteins. This category includes starches as arrowroot, cornstarch, katakuri starch, potato starch, sago, tapioca and their starch derivatives. Vegetable gums used as food thickeners may include alginin, guar gum, locust bean gum, and / or xanthan gum. Exemplary proteins include collagen, egg whites, furcellaran, and / or gelatin. Sugars include agar and carrageenan.

Generally, suitable thickeners may be selected from the group consisting of agar (E 406), alginic acid (E 400), natrium alginate (E 401), potassium alginate (E 402), carrageenan (E 407), locust bean gum (E 410), guar gum (E 412), gum tragacanth (E 413), arabic gum (E 414), xanthan gum (E 415), karaya gum (E 416), tara gum(E 417), gellan gum (E 418), pectins (E 440), cellulose (E 460), celluloseethers (including methyl cellulose (E 461), ethyl cellulose (E 462), hydroxypropyl cellulose (E 463), hydroxypropylmethylcellulose (E 464), methylethylcellulose (E 465), carboxymethylcellulose (E 466)) and modified starches (including oxidised starch (E 1404), mono-starch phosphate (E 1410), di-starch phosphate (E 1412), phosphorylated di-starch phosphate (E 1413), acetylated di-starch phosphate (E 1414), acetylated stach (E 1420), acetylated di-starch adipate (E 1422), hydroxypropylstach (E 1440), hydroxypropyl di-starch phosphate (E 1442), starch sodium octenyl succinate (E 1450), acetylated oxidised starch (E 1451)).

Enteral nutrition products optionally comprise ingredients selected from the group consisting of choline, beta-carotene, lutein, lucopene, caffeine, lecithin, taurine, carnitine, myo-inositol, stabilisers, emulsifiers, colorants, aroma and mixtures thereof. Aromas may be caramel, vanilla, yoghurt, chocolate, coffee, cappuccino or fruit aromas.

At least for safety reasons, the enteral nutrition products comprised by the system herein should preferably be free of x-ray contrast agents, in particular free of BaSO₄.

### METHODS

### Viscosity Profiles

All rheological measurements are to be carried out at 25°C. A controlled-stress rheometer (Modular Advanced Rheometer System MARS, Haake, Thermo-Scientific, Germany), using a serrated plate-and-plate geometry (35 mm diameter, 1 mm gap) may be used. Samples should have had the same recent past thermal and mechanical history. Viscous flow curves were obtained from shear rate sweep tests, in a range of 10-2 - 100 s-1. The viscosity values used for viscosity profiles (i.e. viscosity vs. shear rate) are obtained under steady-state conditions.

All rheological measurements are done in triplicate and averaged.

## Claims

1. System for use in diagnosis of dysphagia and nutrition of dysphagia patients comprising at least a first, a second and a third enteral nutrition product having each
a) a defined nutrient profile balanced for complete enteral nutrition and / or for supplemental enteral nutrition;
b) a shear rate dependent viscosity profile, wherein at each shear rate value between 1-100 s⁻¹ the following condition is fulfilled:
viscosity product 1 > viscosity product 2 > viscosity product 3.

2. System according to claim 1, wherein the diagnosis is endoscopic.

3. System according to claims 1 or 2, further comprising a fourth enteral nutrition product having a nutrient profile balanced for complete enteral nutrition and / or for supplemental enteral nutrition and a shear rate dependent viscosity profile, wherein at each shear rate value between 1 and 100 s⁻¹ fulfils the following condition:
viscosity product 3 > viscosity product 4.

4. System according to any preceding claim, wherein each product has a shear thinning viscosity profile.

5. System according to any preceding claim, wherein the viscosity profiles in a log-log scale are essentially parallel.

6. System according to any of the preceding claims, wherein the viscosity of the first product at 1 s⁻¹ is lower than 10³ and higher than 10² Pas.

7. System according to any of the preceding claims, wherein the viscosity of the second product at 1 s⁻¹ is lower than 10² and higher than 10¹ Pas.

8. System according to any of the preceding claims, wherein the viscosity of the third product at 1 s⁻¹ is lower than 10¹ and higher than 10° Pas higher than the viscosity of the fourth product.

9. System according to any of the preceding claims, wherein the viscosity of each of the products at 1 s⁻¹ lies between 10⁻²-10³ Pas.

10. System according to any of the preceding claims, wherein the viscosity of the first product at 1 s⁻¹ is at least 10 Pas higher than the viscosity of the second product.

## Patentansprüche

1. System zur Verwendung in der Dysphagiediagnose und der Ernährung von Dysphagiepatienten mindestens umfassend ein erstes, ein zweites und ein drittes enterales Ernährungsprodukt, welche jeweils Folgendes aufweisen
a) ein definiertes Nährwertprofil, welches für eine komplette enterale Ernährung und/oder für eine ergänzende enteral Ernährung ausgewogen ist;
b) ein von der Schergeschwindigkeit abhängiges Viskositätsprofil, wobei bei jedem Schergeschwindigkeitswert zwischen 1-100 s⁻¹ die folgende Bedingung erfüllt wird:
Viskosität von Produkt 1 > Viskosität von Produkt 2 > Viskosität von Produkt 3.

2. System nach Anspruch 1, wobei die Diagnose endoskopisch ist.

3. System nach den Ansprüchen 1 oder 2, zusätzlich umfassend ein viertes enterales Ernährungsprodukt aufweisend ein Nährwertprofil, welches für eine komplette enterale Ernährung und/oder für eine ergänzende enterale Ernährung ausgewogen ist, und ein von der Schergeschwindigkeit abhängiges Viskositätsprofil, wobei bei jedem Schergeschwindigkeitswert zwischen 1 und 100 s⁻¹ die folgende Bedingung erfüllt wird:
Viskosität von Produkt 3 > Viskosität von Produkt 4.

4. System nach einem vorhergehenden Anspruch, wobei jedes Produkt ein scherverdünnendes Viskositätsprofil aufweist.

5. System nach einem vorhergehenden Anspruch, wobei die Viskositätsprofile in einer Log-Log-Skala im Wesentlichen parallel sind.

6. System nach einem der vorhergehenden Ansprüche, wobei die Viskosität des ersten Produkts bei 1 s⁻¹ niedriger als 10³ und höher als 10² Pas ist.

7. System nach einem der vorhergehenden Ansprüche, wobei die Viskosität des zweiten Produkts bei 1 s⁻¹ niedriger als 10² und höher als 10¹ Pas ist.

8. System nach einem der vorhergehenden Ansprüche, wobei die Viskosität des dritten Produkts bei 1 s⁻¹ weniger als 10¹ und mehr als 10° Pas höher als die Viskosität des vierten Produkts ist.

9. System nach einem der vorhergehenden Ansprüche, wobei die Viskosität von jedem der Produkte bei 1 s⁻¹ zwischen 10⁻²-10³ Pas liegt.

10. System nach einem der vorhergehenden Ansprüche, wobei die Viskosität des ersten Produkts bei 1 s⁻¹ mindestens 10 Pas höher als die Viskosität des zweiten Produkts ist.

## Revendications

1. Système destiné à être utilisé dans un diagnostic de dysphagie et dans la nutrition chez des patients atteints de dysphagie comprenant au moins un premier, un second et un troisième produit de nutrition entérale, chacun ayant
a) un profil nutritionnel défini équilibré pour nutrition entérale complète et/ou pour nutrition entérale supplémentaire ;
b) un profil de viscosité dépendant du taux de cisaillement, dans lequel à chaque valeur du taux de cisaillement entre 1 - 100 s⁻¹, la condition suivante est satisfaite :
viscosité de produit 1 > viscosité de produit 2 > viscosité de produit 3.

2. Système selon la revendication 1, dans lequel le diagnostic est endoscopique.

3. Système selon les revendications 1 ou 2, comprenant en outre un quatrième produit de nutrition entérale ayant un profil nutritionnel équilibré pour nutrition entérale complète et/ou pour nutrition entérale supplémentaire et un profil de viscosité dépendant du taux de cisaillement, dans lequel à chaque valeur du taux de cisaillement entre 1 et 100 s⁻¹, la condition suivante est satisfaite :
viscosité de produit 3 > viscosité de produit 4.

4. Système selon l'une quelconque des revendications précédentes, dans lequel chaque produit a un profil de viscosité à fluidification par cisaillement.

5. Système selon l'une quelconque des revendications précédentes, dans lequel les profils de viscosité dans une échelle log-log sont essentiellement parallèles.

6. Système selon l'une quelconque des revendications précédentes, dans lequel la viscosité du premier produit à 1 s⁻¹ est inférieure à 10³ et supérieure à 10² Pas.

7. Système selon l'une quelconque des revendications précédentes, dans lequel la viscosité du second produit à 1 s⁻¹ est inférieure à 10² et supérieure à 10¹ Pas.

8. Système selon l'une quelconque des revendications précédentes, dans lequel la viscosité du troisième produit à 1 s⁻¹ est inférieure à 10¹ et supérieure à 10° Pas supérieure à la viscosité du quatrième produit.

9. Système selon l'une quelconque des revendications précédentes, dans lequel la viscosité de chacun des produits à 1 s⁻¹ se trouve entre 10⁻² - 10³ Pas.

10. Système selon l'une quelconque des revendications précédentes, dans lequel la viscosité du premier produit à 1 s⁻¹ est au moins 10 Pas supérieure à la viscosité du second produit.
